# EUROPEAN PATENT APPLICATION

(11) **EP 4 843 501 A1**
(43) Date of publication of application: **25.11.2026**
(21) Application number: 25178272.8
(22) Date of filing: 22.05.2025
(51) Int. Cl.: A61B 5/287, A61B 5/00, A61B 18/14

(54) **HIGH DENSITY ELECTRODE MAPPING CATHETER AND METHOD OF USE**

(71) Applicant: AF-ABLATION S.R.L., 20121 Milan (MI) (IT)
(72) Inventor: PAPPONE, Carlo, Milan (IT)
(74) Representative: Bird & Bird Società tra Avvocati S.r.l.

(57) **Abstract**

The innovative catheter comprises an high-density mapping catheter (1000), wherein the catheter (1000) includes: an electromagnetic tracking sensor (250) disposed in a manifold (210) of a middle section (200) of the catheter (1000) and a mapping device (100) disposed in a distal portion of the catheter (1000), wherein a mapping device (100) comprising: a central strip column (110), a plurality of railings (120) arranged symmetrically and parallel to the central strip column (110); a plurality of electrodes (130) intimately connected to the plurality of railings (120) through a pass-through connection. Therefore, an innovative method (2000) of use of a high-density mapping catheter with an innovative step of inserting and anchoring a plurality of electrodes into a slot of a flexible internal structural support of a plurality of flexibly railings.

## Description

### TECHNICAL FIELD

The subject-matter disclosed herein relates to high-density mapping catheter of a cardiac tissue and method of use.

### BACKGROUND ART

Catheters have been integral to cardiac electrophysiology for decades, enabling both diagnostic and therapeutic interventions in regions of the heart that are otherwise inaccessible without open surgical procedures. These devices are commonly used to diagnose and treat cardiac arrhythmias by delivering electrical signals or ablative energy to targeted myocardial tissue.

Conventional cardiac mapping catheters typically feature a series of ring electrodes arranged circumferentially around the catheter shaft, often fabricated from biocompatible conductive materials such as platinum-iridium alloys. These electrodes are generally rigid and non-deformable, which can limit their ability to conform to the complex three-dimensional geometry of the endocardial or epicardial surface. Similarly, standard ablation catheters include a rigid distal tip electrode designed to deliver radiofrequency (RF) energy for lesion formation and may also incorporate ring electrodes for electrogram acquisition.

However, the rigidity of these electrodes poses significant challenges in achieving and maintaining consistent tissue contact, particularly in anatomically complex regions such as the atrial appendages, trabeculated ventricular surfaces, or near valve annuli. Poor electrode-tissue contact can result in low signal fidelity, inaccurate electro anatomical mapping, and ineffective lesion formation due to suboptimal energy delivery.

These limitations are further exacerbated by the dynamic nature of the beating heart. Cardiac motion, especially in the presence of arrhythmias such as atrial fibrillation or ventricular tachycardia, introduces variability in catheter-tissue contact. This variability can compromise the stability of electrogram recordings and the precision of ablation. On highly contoured or trabeculated surfaces, maintaining stable contact for the duration required to acquire high-resolution maps or deliver effective lesions becomes particularly difficult.

Advanced mapping technologies, such as those employing high-density microelectrode arrays, have been developed to improve spatial resolution and signal acquisition. These catheters often feature multiple closely spaced microelectrodes capable of detecting localized and focal electrical potentials with greater precision. However, despite their enhanced resolution, these systems are not without limitations. The small size and high density of microelectrodes can lead to signal oversampling, increased noise susceptibility, and difficulty in distinguishing true local electrograms from far-field signals or artifacts. Additionally, the interpretation of microelectrode data requires sophisticated signal processing algorithms and expert clinical judgment, particularly when attempting to identify low-amplitude, fractionated, or transient potentials associated with arrhythmogenic substrates.

Moreover, the limited depth of field of microelectrode recordings may hinder the detection of intramural or epicardial sources of arrhythmia, especially in thick-walled cardiac structures. As such, while high-density mapping catheters represent a significant advancement in electrophysiology, their diagnostic utility must be carefully balanced against these technical constraints.

Several patented technologies, including those described in US Patent No. 11,183,078 and US Patent No. 11,039,773, propose catheter designs with improved electrode configurations or flexible structures aimed at enhancing contact and mapping fidelity. However, these solutions still rely on relatively rigid or semi-rigid electrode architectures that do not fully conform to the highly variable and dynamic topography of the epicardial surface. As a result, they fall short in enabling true adaptability to complex anatomical contours and do not provide the consistent, high-resolution electrogram acquisition necessary for precise identification of localized and focal potentials.

Additionally, the noted device QDOT Micro^{™} catheter by Biosense Webster, is not able to offer flexible multi-railing structure and adaptability to irregular epicardial surfaces; stable anchoring of micro-electrodes; improved signal quality during cardiac motion.

Furthermore, these designs often lack the capability to perform real-time, high-density electrocardiographic mapping with sufficient spatial and temporal resolution to resolve micro-reentrant circuits or pinpoint discrete arrhythmogenic foci. Consequently, while these patents represent incremental improvements, they do not fully address the fundamental limitations of current catheter technologies in achieving optimal tissue contact, signal clarity, and mapping accuracy on the epicardial surface.

The preceding discussion highlights the ongoing evolution in the field of catheter-based cardiac interventions and underscores the need for further innovation in catheter design, electrode placement and fixation within the catheter, and overall flexibility. Such advancements are essential to overcome the inherent limitations of current technologies and to enable truly adaptive, high-resolution epicardial mapping and therapy.

### SUMMARY

It would be desirable to have an innovative catheter comprises a high-density mapping catheter, wherein the catheter includes: an electromagnetic tracking sensor disposed in a manifold of a middle section of the catheter and a mapping device disposed in a distal portion of the catheter, wherein the mapping device comprising: a central strip column; a plurality of railings arranged symmetrically and parallel to the central strip column ; a plurality of electrodes intimately connected to the plurality of railings through a pass-through connection.

The invention further relates to an innovative method of use of a high-density mapping catheter with an innovative step of inserting and anchoring a plurality of electrodes into a slot of a flexible internal structural support of a plurality of flexibly railings.

### BRIEF DESCRIPTION OF THE DRAWINGS

A more complete appreciation of the disclosed embodiments of the invention and many of the attendant advantages thereof will be readily obtained as the same becomes better understood by reference to the following detailed description when considered in connection with the accompanying drawings, wherein:
Fig. 1 shows a prospective view of a first embodiment of an innovative catheter.
Fig. 1A shows a prospective view of a second embodiment of an innovative catheter;
Figs. 2 and 2a show prospective view of a internal structure of a component of the innovative catheter of Fig. 1;
Fig. 3 shows a prospective view of a third embodiment of an innovative catheter. of Fig. 1;

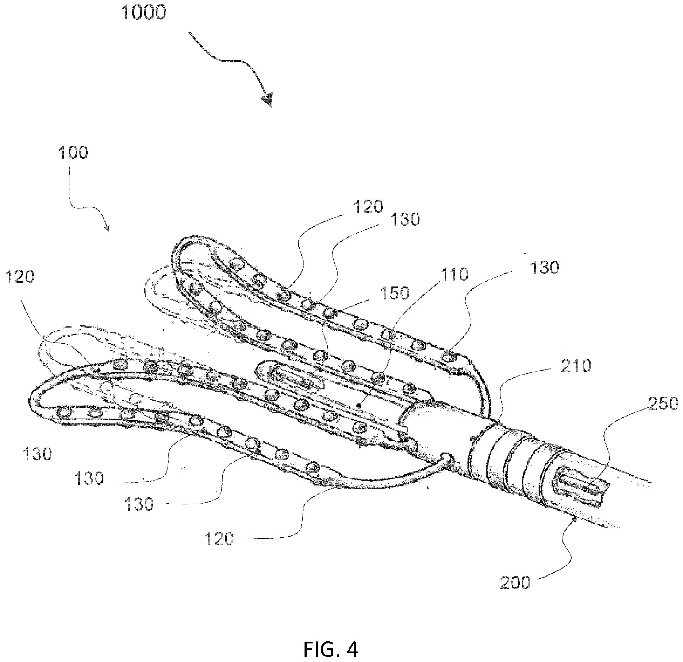

Fig. 4 shows a side elevation view thereof in a flexed position of the innovative catheter of Fig. 3;
Figs. 5, and 6 show a prospective view of a detailed features of an electrode inserted in a rail of the innovative catheter of Fig. 1;
Figs. 7A, 7B and 7C show a view of embodiments of an electrode of the catheter of the invention;

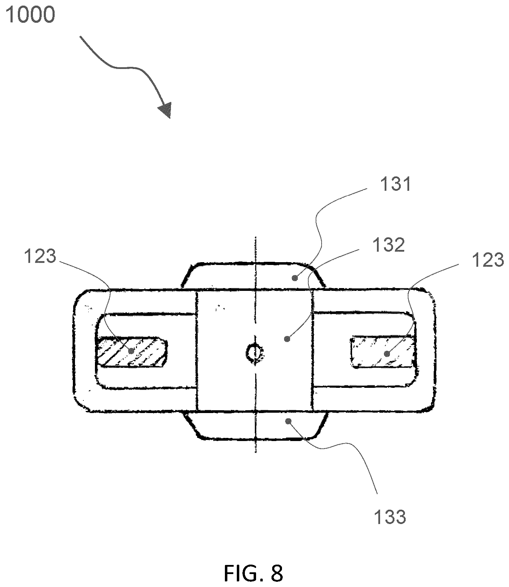

Fig.8 shows a cross-section view of a flexible rail with electrode in place in the innovative catheter of Fig. 1;
Fig. 9 show prospective view a rectangular shape of a plurality electrodes of the innovative catheter of Fig. 1;
Fig. 10 shows a flow chart of an embodiment of an innovative method of use of the catheter of Fig. 1;
Fig. 11 shows a flow chart of an embodiment of a step of an innovative method of use of the catheter of Fig. 1.

### DETAILED DESCRIPTION OF EMBODIMENTS

The high-density mapping catheter of invention introduces a novel architecture designed to enhance both adaptability and signal fidelity during cardiac procedures. At its core, the catheter integrates an electromagnetic tracking sensor positioned within the manifold of the middle section, enabling precise spatial localization during navigation. Toward the distal end, the catheter features a sophisticated mapping device composed of a central strip column flanked symmetrically by multiple flexible railings. These railings run parallel to the central column and serve as the structural foundation for a series of electrodes, which are securely mounted through a pass-through connection system.

What sets this catheter apart is the flexibility and intelligent design of its railings. Each railing incorporates an internal structural support that includes a longitudinal slot, allowing the electrodes to be inserted and fixed with precision. This configuration not only ensures mechanical stability but also facilitates intimate and consistent contact between the electrodes and the epicardial surface. Such contact is critical for acquiring high-fidelity electrograms and delivering effective ablation therapy, particularly in anatomically complex or dynamically moving regions of the heart.

The internal supports are insulated with high-dielectric materials, enhancing electrical isolation and safety while maintaining the flexibility required for the catheter to conform to the heart's variable topography. Additionally, the presence of flexible holes dimensionally matched to the internal slots further simplifies electrode integration, ensuring secure placement without compromising the catheter's adaptability.

This design offers several clinical advantages. By maintaining stable electrode-tissue contact across irregular surfaces, it improves the accuracy of electro anatomical mapping and supports high-resolution signal acquisition. The flexible structure also reduces the risk of mechanical trauma and enhances maneuverability within the cardiac chambers. Overall, this catheter represents a significant step forward in enabling precise, adaptive, and high-resolution epicardial mapping and therapy

Reference now will be made in detail to embodiments of the disclosure, an example of which is illustrated in the drawings. Each example is provided by way of explanation of the disclosure, not limitation of the disclosure. In fact, it will be apparent to those skilled in the art that various modifications and variations can be made in the present disclosure without departing from the scope or spirit of the disclosure. In the following description, similar reference numerals are used for the illustration of figures of the embodiments to indicate elements performing the same or similar functions. Moreover, for clarity of illustration, some references may not be repeated in all the figures.

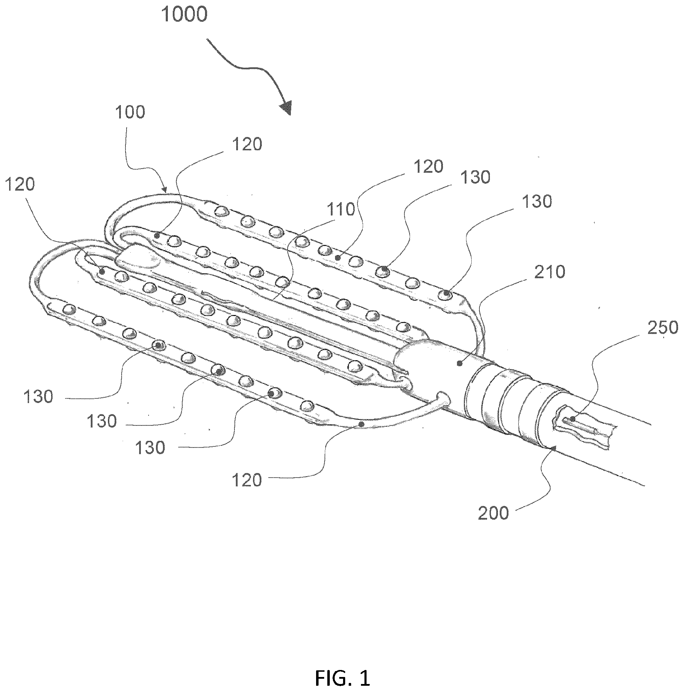

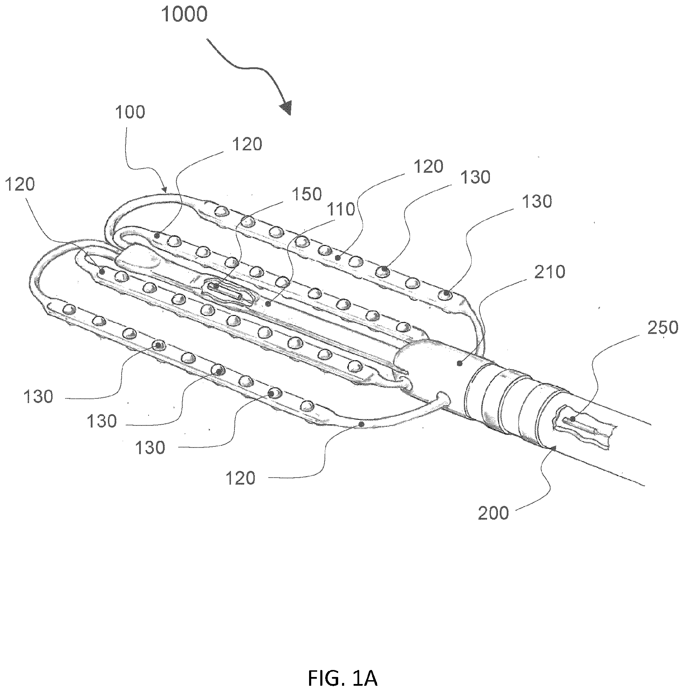

Fig. 1 schematically shows one embodiment of an innovative catheter equipped with a mapping device. The innovative catheter is generally indicated with reference numeral 1000 in Fig. 1.

With non-limiting reference to Fig. 1 the catheter 1000 is a flexible tubular shaft that includes a proximal section equipped with handle, a middle section 200, and a distal section that are connected in sequence.

Considering Fig. 1 the catheter comprises a catheter 1000, wherein the catheter 1000 includes: an electromagnetic tracking sensor 250 disposed in a manifold 210 of a middle section 200 of the catheter 1000 and a mapping device 100 disposed in a distal section of the catheter 1000, wherein the mapping device 100 is connected to the middle section 200 through a manifold 210.

Advantageously the mapping device 100 comprising: a central strip column 110, a plurality of railings 120 arranged symmetrically and parallel to the central strip column 110; and a plurality of electrodes 130 intimately connected to the plurality of railings 120 through a pass-through connection.

Reference to Fig. 1 the manifold 210 is able to connect a distal portion of each of the plurality of flexible railings 120, and distal portion of the central strip column 110.

The plurality of flexible railings 120 is arranged parallel to each other and positioned longitudinal to the axis of the catheter shaft. The plurality of flexible railings has a rectangular or flat shape or plurality of round filar or a combination of shapes of high memory material such as NiTi or equivalent to facilitate flexing vertically bi-directional or radial motion, to conform to epicardium surfaces, to move in-sync with cardiac motion and minimize horizontal or lateral curve.

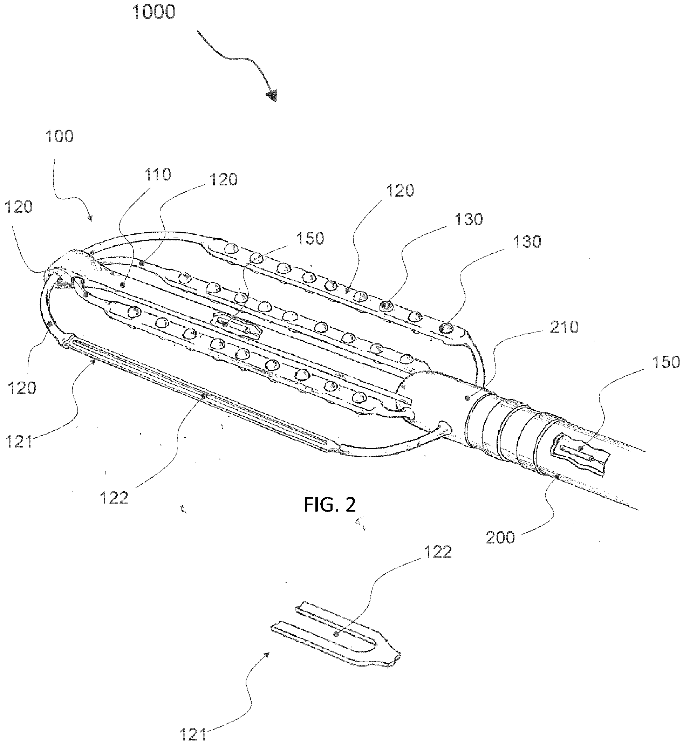

According to preferred embodiments (see e.g. Fig. 2) the plurality of railings 120 is flexible railings, and comprises at least an internal structure support 121, wherein at least the internal structure support 121 is provided with a slot 122 arranged longitudinally and centrally along each of the plurality of railings 120. The flexible railings 120 adapt to the epicardial surface, maintaining consistent electrode-tissue contact, which is essential for accurate electrogram acquisition and effective ablation.

Advantageously at least the internal structure support 121 is flexible and insulated with high dielectric insulation such as polyimide or equivalent, facilitating the insertion and fixing of each of the plurality of electrodes 130.

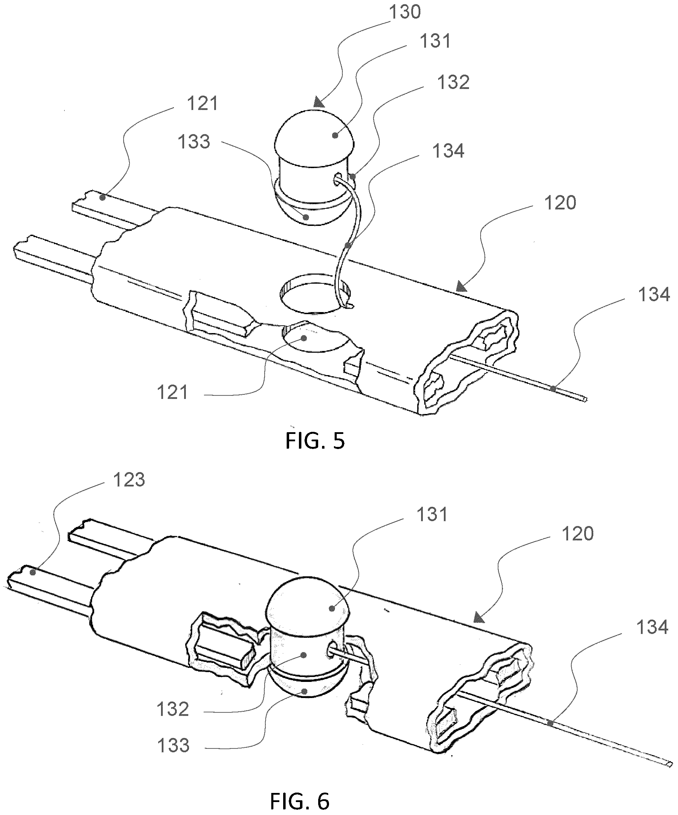

Figures 2, 2A and 5 show for example and without limitation that the plurality railings 120 comprises at least a flexible hole 123 dimensionally comparable to the physical size of the slot 122 of the internal structure support 121, facilitating the insertion and fixing of each of the plurality of electrodes.

It is to be noted that the pass-through connection is the internal structure support 121 of the plurality of railings 120.

Turning now to the Figures each of the plurality of electrodes 130 comprises an upper portion 131, a central portion 132, and a lower portion 133 wherein the upper portion and the lower portion have larger physical dimensions than the central portion to locking central portion of each of the plurality of electrodes 130 in the slot 122 of the internal structure support 121.

As shown in Figs, 5 and 6 the upper portion 131 and the lower portion protrude outward relative to the internal structure support 121 of the plurality of railings 120, when each of the plurality of electrodes is connected to the plurality of railings 120.

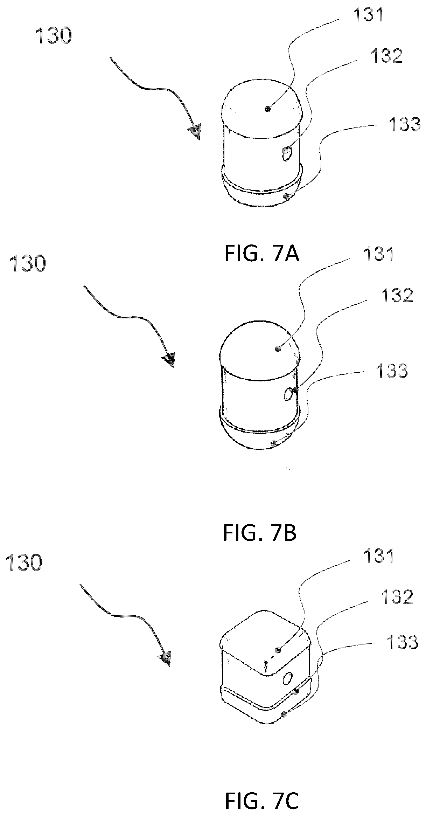

Cosidering Figs 7A, 7B, and 7C, the upper portion 131 and the lower portion 133 are a round portion, or a truncated dome portion, or a truncated square portion. The micro-electrodes are designed with undercut geometry that ensures stable anchoring to the railings, preventing displacement during cardiac motion and preserving signal quality.

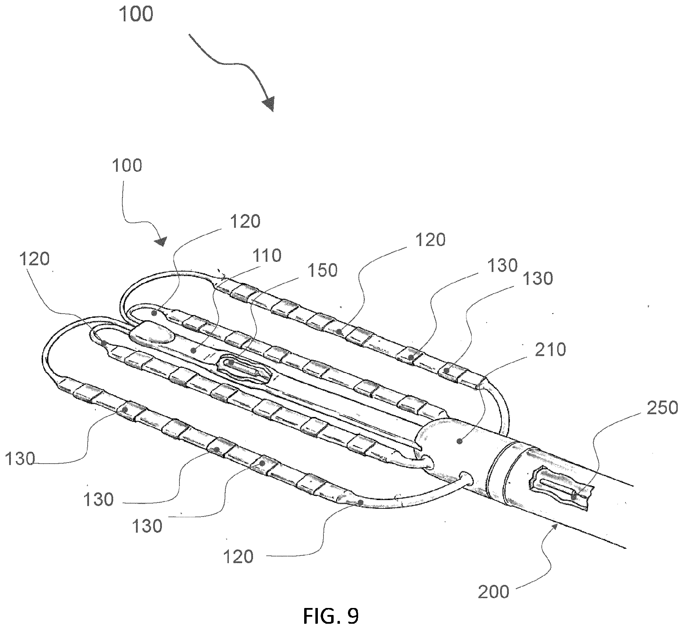

Moreover, wherein the plurality of electrodes 130 has a circular or rectangular shape as shown in Figs. 1 and 9 and preferably is disposed along each of the plurality of railings 120 in a linear configuration.

As shown in Fig, 5 and 6 Advantageously the plurality of electrodes 130 is mutually and electrically connected by at least a conductor wire 134, hereby forming an extended electrode to deliver electrical activity and means of safety line.

The plurality of electrodes is designed with undercut geometry that ensures stable anchoring to the railings, preventing displacement during cardiac motion and preserving signal quality.

As shown in Figs 5 and 6, the extended electrode is designed with "undercut" (meaning the middle section of the electrode is smaller than the top and bottom section). Therefore, when each of the plurality of the electrodes is pressed fit through the hole 123 on the flexible rail assembly, each of the plurality of the electrodes is captured and secured in place.

Advantageously the plurality of electrodes 1130 is micro-electrodes buttons and is equidistant and/or variable disposed along the plurality of railings 120, enabling high-resolution mapping by capturing localized near-field signals while minimizing far-field interference.

The configuration of the plurality of micro-electrode buttons arranged along flexible plurality of railings. Enables to:
- reduction of far-field signals: the close arrangement of the micro-electrodes minimizes interference from distant signals, improving near-field signal quality;
- high-resolution mapping: the density and layout of the micro-electrodes allow for detailed mapping of cardiac areas;
- adaptability to epicardial surfaces: the flexible railings conform to epicardial irregularities, ensuring optimal contact.

Preferably optimal size of the button electrode is like diameter a value d with 0.6mm < d < 1.5mm and like height a value h with 0.2mm < h < 0.5mm.

According to a first embodiment shown in Fig. 1 a proximal portion of each of the plurality of flexible railings 120 are connected with a proximal portion of the central strip column 110.

According to a second embodiment shown in Fig. 2A the central strip column 110 comprises a second electromagnetic tracking sensor 150 to allow precise tracking of the catheter's position, facilitating real-time navigation and mapping. Advantageously The tracking sensor provides precise location of distal section of the catheter that holds the plurality of railings with the plurality of electrodes through anatomical space that are difficult to view and/or access.

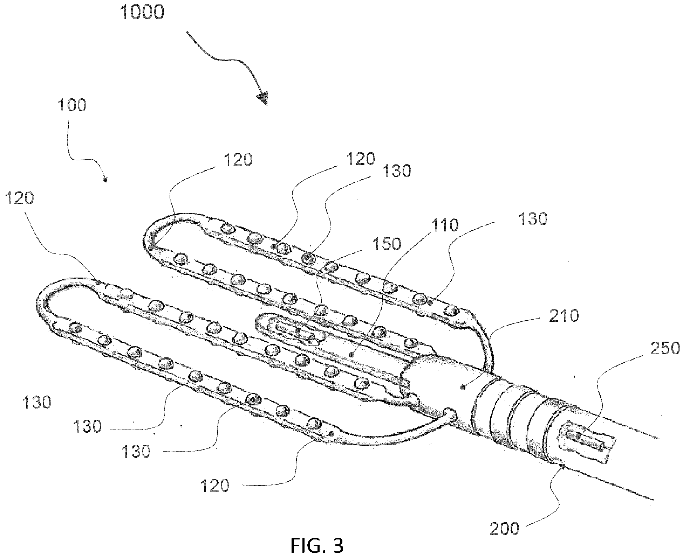

According to a third embodiment shown in Fig. 3 a proximal portion of two or more of the plurality of flexible railings 120 are interconnected.

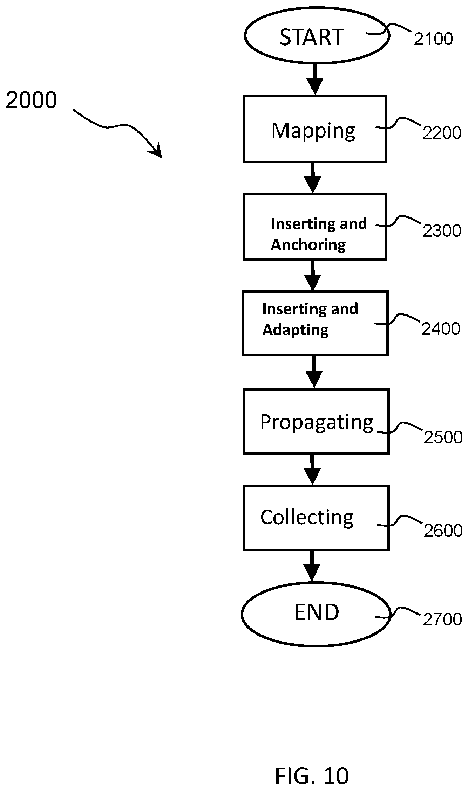

Fig. 10 illustrates a flow chart 2000 of an innovative method of use of a high-density mapping catheter, wherein the catheter is able to map a cardiac tissue of a patient to be treated. The flow chart has a start block 2100 and an end block 2700; the steps corresponding to block 2200 to block 2600 are typically repeated several times. Advantageously, the method 2000 comprises the steps of:
- identifying 2200 a target area of a cardiac tissue to be mapped;
- inserting and anchoring 2300 a plurality of electrodes long away a plurality of railings of the catheter;
- inserting and adapting 2400 of the catheter (preferably considering Fig.s 7A -7CB, is able to map) into the target area;
- propagating 2500 electrical impulses in the target area through the plurality of electrodes on the plurality of railings of the catheter;
- collecting 2600 electrical signals and performing the corresponding mapping.

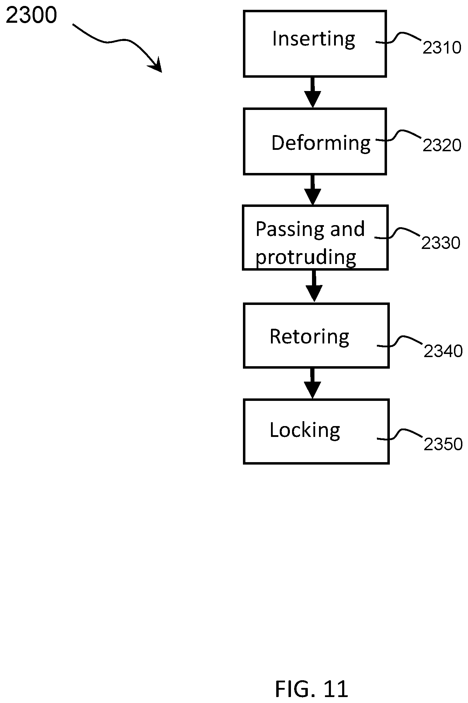

Furthermore Fig. 11 shows that the step of inserting and anchoring 2300 comprises the steps of:
- inserting 2310 by pressing each of the plurality of electrodes into a slot of a flexible internal structural support of the plurality of railings;
- deforming elastically 2320 the slot in the flexible internal structural support of the plurality of railings;
- passing through and protruding 2330 the lower portion of each of the plurality of electrodes through the slot of the internal structural support, and positioning the central portion within the slot of the internal structural support;
- restoring 2340 the original shape of the internal structural support.

locking 2350 by protruding the upper and lower portions of each of the plurality of electrodes outwardly, both above and below, with respect to the internal structural support.

Advantageously, the step of propagating 2500 is carried out by modulating the intensity and frequency of the impulses emitted by the plurality of electrodes 130 of the plurality of railings 120, through an impulse generator capable of switching between emitting short pulses of high-voltage energy and pulses of low-voltage energy, and vice versa.

The high-density mapping catheter described herein is suitable for a variety of electrophysiological applications including but not limited to:
- mapping of atrial fibrillation substrate to characterize low-voltage, fractionated, or non-pulmonary vein-related regions in persistent atrial fibrillation, including the posterior wall, anterior line, and septum;
- mapping of arrhythmogenic substrate in cardiogenetic syndromes, including Brugada syndrome, arrhythmogenic right ventricular cardiomyopathy (ARVC), Long-QT Syndrome and early repolarization syndrome, especially in epicardial locations;
- characterization of epicardial fat and adipose tissue regions, e.g. to differentiate epicardial adipose tissue from working myocardium, particularly relevant in atrial fibrillation and Brugada syndrome. Additionally, the catheter of the invention may assist in mapping and characterizing epicardial adipose tissue in patients with heart failure with preserved ejection fraction (HFpEF), where the metabolic, inflammatory, and fibrotic roles of this tissue are gaining increasing role as potential contributors to diastolic dysfunction and arrhythmogenesis;
- mapping of inflammatory or infiltrative cardiomyopathies, such as electro-anatomical characterization of arrhythmogenic zones in conditions like cardiac sarcoidosis and myocarditis;
- epicardial mapping of ventricular tachycardia substrate, such as for high-resolution mapping of post-infarction or non-ischemic scar regions to identify slow-conduction isthmuses and critical VT circuits;
- intramural or septal mapping in complex ventricular arrhythmias, including mapping of deep intramural foci at the left ventricular summit or within the interventricular septum using enhanced electrode-tissue contact;
- substrate mapping in congenital or post-surgical heart diseases, e.g. for patients with complex anatomy (e.g., post-Fontan, tetralogy of Fallot), where adaptability to non-uniform cardiac surfaces is critical;
- functional mapping techniques (e.g., Isochronal Late Activation Mapping, Phase Mapping) enabling detection of conduction delay zones and micro-reentry using high-density electrogram acquisition;
- electrophysiological mapping in onco-cardiology, such as for assessment of electrical activity in regions affected by cardiac tumors or radiation-induced fibrosis;
- validation and post-ablation assessment of lesion integrity, e.g. to verify the continuity and completeness of ablation lesions, detect conduction gaps, and assess endpoint criteria, particularly in atrial fibrillation and ventricular ablation procedures.

## Claims

1. A high-density mapping catheter (1000),
wherein the catheter (1000) includes: an electromagnetic tracking sensor (250) disposed in a manifold (210) of a middle section (200) of the catheter (1000) and a mapping device (100) disposed in a distal portion of the catheter (1000),
wherein the mapping device (100) comprising:
- a central strip column (110):
- a plurality of railings (120) arranged symmetrically and parallel to the central strip column (110);
- a plurality of electrodes (130) intimately connected to the plurality of railings (120) through a pass-through connection.

2. The catheter (1000) of claim 1, wherein the plurality of railings (120) is flexible railings, and comprises at least an internal structure support (121), wherein at least the internal structure support (121) is provided with a slot (122) arranged longitudinally and centrally along each of the plurality of railings (120).

3. The catheter (1000) of claim 2, wherein at least the internal structure support (121) is flexible and insulated with high dielectric insulation, facilitating the insertion and fixing of each of the plurality of electrodes (130).

4. The catheter (1000) of claim 1, wherein each of the plurality railings (120) comprises at least a flexible hole (123) dimensionally comparable to the physical size of the slot (122) of the internal structure support (121), facilitating the insertion and fixing of each of the plurality of electrodes.

5. The catheter (1000) of claims 1 and 2, wherein the pass-through connection is the internal structure support (121) of the plurality of railings (120).

6. The catheter (1000) of claim 1 and 2, wherein each of the plurality of electrodes (130) comprises an upper portion (131), a central portion (132), and a lower portion (133) wherein the upper portion and the lower portion have larger physical dimensions than the central portion to locking central portion of each of the plurality of electrodes (130) in the slot (122) of the internal structure support (121).

7. The catheter (1000) of claim 6, wherein the upper portion (131) and the lower portion protrude outward relative to the internal structure support (121) of the plurality of railings (120), when each of the plurality of electrodes is connected to the plurality of railings (120).

8. The catheter (1000) of claim 2, wherein the upper portion (131) and the lower portion (133) are a round portion, or a truncated dome portion, or a truncated square portion.

9. The catheter (1000) of claim 1, wherein the plurality of electrodes (130) has a circular or rectangular shape.

10. The catheter (1000) of claim 1, wherein the plurality of electrodes (130) is disposed along each of the plurality of railings (120) in a linear configuration.

11. The catheter (1000) of claim 1, wherein the plurality of electrodes (130) are mutually and electrically connected by at least a conductor wire (134).

12. The catheter (1000) of claim 1, wherein the plurality of electrodes (130) is micro-electrodes buttons.

13. The catheter (1000) of claim 1, wherein the plurality of electrodes (130) is equidistant and/or variable disposed along the plurality of railings (120).

14. The catheter (1000) of claim 1, wherein the plurality of flexible railings has a rectangular and/or flat shape.

15. The catheter (1000) of claim 1, wherein a proximal portion of each of the plurality of flexible railings (120) are connected with a proximal portion of the central strip column (110).

16. The catheter (1000) of claim 1 wherein a proximal portion of two or more of the plurality of flexible railings (120) are interconnected.

17. The catheter (1000) of claim 1, wherein the manifold (210) is able to connect a distal portion of each of the plurality of flexible railings (120), and distal portion of the central strip column (110).

18. The catheter (1000) of claim 1, wherein the central strip column (110) comprises a second electromagnetic tracking sensor (150).

19. A method (2000) of use of a high-density mapping catheter according to anyone of claims 1 to 18, wherein the high-density mapping catheter is able to mapping a cardiac tissue of a patient to be treated, wherein the method (2000) comprising the steps of:
- identifying (2200) a target area of a cardiac tissue to be mapped;
- inserting and anchoring (2300) a plurality of electrodes long away a plurality of railings of the catheter;
- inserting and adapting (2400) of the catheter into the target area;
- propagating (2500) electrical impulses in the target area through the plurality of electrodes on the plurality of railings of the catheter; and
- collecting (2600) electrical signals and performing the corresponding mapping.

20. The method (2000) of claim 19, wherein the step of inserting and anchoring (2300) comprises the steps of:
- inserting (2310) by pressing each of the plurality of electrodes into a slot of a flexible internal structural support of the plurality of railings;
- deforming elastically (2320) the slot in the flexible internal structural support of the plurality of railings;
- passing through and protruding (2330) the lower portion of each of the plurality of electrodes through the slot of the internal structural support, and positioning the central portion within the slot of the internal structural support;
- restoring (2340) the original shape of the internal structural support; and
- locking (2350) by protruding the upper and lower portions of each of the plurality of electrodes outwardly, both above and below, with respect to the internal structural support.

21. The method (2000) of claim 19, wherein the step of propagating (2500) is carried out by modulating the intensity and frequency of the impulses emitted by the plurality of electrodes (130) of the plurality of railings (120), through an impulse generator capable of switching between emitting short pulses of high-voltage energy and pulses of low-voltage energy, and vice versa.
